# EUROPÄISCHE PATENTSCHRIFT

(11) **EP 0 848 960 B1**
(45) Veröffentlichungstag und Bekanntmachung des Hinweises auf die Patenterteilung: **24.04.2002**
(21) Anmeldenummer: 97120916.8
(22) Anmeldetag: 28.11.1997
(51) Int. Cl.: A61L 15/58, C09J 133/06

(54) **Haft- und Bindemittel für dermale oder transdermale Therapiesysteme**
Adhesive for dermal or transdermal therapy systems
Adhésif pour système thérapeutique dermique ou transdermique

(30) Priorität: 20.12.1996 DE 19653605
(43) Veröffentlichungstag der Anmeldung: 24.06.1998
(73) Patentinhaber: Röhm GmbH & Co. KG, 64293 Darmstadt (DE)
(72) Erfinder: Assmus, Manfred, 64404 Bickenbach (DE); Beckert, Thomas, Dr., 64297 Darmstadt (DE); Bergmann, Günter, 64435 Gross Krotzenburg (DE); Kähler, Stephanie, 64625 Bensheim (DE); Petereit, Hans-Ulrich, 64291 Darmstadt (DE)

(56) Entgegenhaltungen:
- EP-A- 0 617 972
- DE-A- 4 429 791

## Beschreibung

Die Erfindung betrifft ein Haft- und Bindemittel für dermale oder transdermale Arzneimittel.

### Stand der Technik

EP-B 415 055 betrifft wasserlösliche druckempfindliche Hauthaftkleber. Diese bestehen dem Salz eines unvernetzten Copolymers aus einem animogruppenhaltigen, monoäthylenisch ungesättigten, radikalisch polymerisierbaren Monomer und wenigstens einem Alkylester der Acrylund/oder Methacrylsäure. Die Formulierung ist dadurch gekennzeichnet, daß sie das Salz wenigstens einer höheren organischen Carbonsäure mit 8 - 20 Kohlenstoffatomen oder eines Gemisches einer solchen höheren Carbonsäure mit bis zu 30 Mol-% (der anionischen Äquivalente) an mittleren Carbonsäuren ist und einen Anteil des aminogruppenhaltigen Monomers im Bereich von 30 - 80 Gew.-% (bezogen auf das Gewicht des Copolymers} enthält und in der Salzform in Wasser löslich ist.

US-PS 3 321 451 beschreibt abwaschbare Hauthaftkleber auf der Basis von Aminogruppen-haltigen (Meth)acrylat-Copolymeren, wobei die Aminogruppen teilweise als Salz eines Säureanions vorliegen.

EP-A 164 669 beschreibt ein Verfahren zum Überziehen von Arzneiformen mittels (Meth)acrylat-Copolymere, die Monomere mit tertiären Aminogruppen enthalten, wobei diese mittels Mineralsäuren oder organischer Säuren, wie z. B. Essigsäure oder Citronensäure in die Salzform überführt werden können. Die Überzüge sollen möglichst wenig klebrig sein, um ein Verkleben der Arzneiformen zu vermeiden.

EP-A 354 364 beschreibt die Verwendung von Aminogruppen enthaltenden Copolymeren in wässriger Zubereitung als Klebstoffe. Die enthaltenen Aminogruppen sind teilweise durch Säuren wie z. B. Ameisensäure oder Essigsäure neutralisiert.

EP-A 315 218 beschreibt pharmazeutische Zusammensetzungen zur transdermalen systemischen Verabreichung von pharmakologischen Wirkstoffen, dadurch gekennzeichnet, daß sich die pharmakologischen Wirkstoffe in einem Reservoir befinden, das ein Polyacrylatpolymer mit kationischen Eigenschaften enthält. Additve wie Weichmacher oder Tenside können in Mengen bis zu 50 Gew.-% enthalten sein. Die pharmazeutische Zusammensetzung kann zusätzlich mit einer Klebeschicht versehen werden, um eine gute Haftung auf der Haut zu erreichen.

EP-A 617 972 beschreibt schichtförmige dermale therapeutische Systeme mit verzögerter Wirkstoffabgabe, die aus Mischungen von Poly(meth)acrylaten bestehen und aus einer Schmelze hergestellt werden. Dabei enthält eine Poly(meth)acrylat-Komponente funktionelle Gruppen, während eine weitere Poly(meth)acrylat-Komponente keine oder nur unerhebliche Mengen an funktionellen Gruppen enthält und im wesentlichen das Fließverhalten der polymeren Klebeschicht reguliert.

### Aufgabe und Lösung

Haft- und Bindemittel für pharmazeutische Zwecke, welche auf (Meth)acrylatcopolymeren mit basischen Gruppen basieren in Kombination mit Weichmachern und mit säuregruppenhaltigen (Meth)acrylatcopolymeren ist im Prinzip aus EP-A 617 972 bekannt.

Ein grundsätzliches Problem bei pharmazeutischen Haft- und Bindemitteln ist die Wasserdampfdurchlässigkeit. Ist diese nicht ausreichend, so ist die Verträglichkeit auf der Haut beeinträchtigt. Außerdem besteht die Gefahr, daß die Präparationen, z. B. Hauthaftpflaster zu schnell eintrocknen, wodurch die kontrollierte Wirkstoffabgabe nachteilig beeinflußt wird.

Die Bestimmung der Haftkraft erfolgt Abziehen eines 50mm breiten, mit dem Haft- und Bindemittel beschichteten Streifens, bevorzugt aus Aluminium, von einer VA-Stahlplatte bei gleichzeigiger Messung der dafür nötigen Kraft. Die Methode ist angelehnt an das Europäische Arzneibuch, (Peel Methode).

Die für Pflaster notwendige Mindesthaftkraft kann entsprechend dem Anwendungszweck unterschiedlich definiert werden. Erfindungsgemäß iegt dieser Wert bei mindestens 10 N/50mm.

Eine weiteres Kriterium stellt der sogenannte Kaltfluß dar. Unter dem Kaltfluß eines Klebers versteht man eine mangelnde Haftung bei einer Krafteinwirkung parallel zur Klebefläche. Auf der menschlichen Haut bedeutet zu hoher Kaltfluß, daß ein Pflaster während des Tragens wandert und dunkle Ränder hinterläßt oder Falten wirft. Dies ist insbesondere bei wirkstoffhaltigen Kleberschichten in transdermalen Therapiesystemen von Nachteil, weil dadurch die Wirkstoffaufnahme in den Körper unkontrollierbar beeinflußt wird.

Der Kaltfluß wird in der Technik nach definierten Konventionsmethoden z.B. PSTC-7 bzw. AFERA 4012-P1 gemessen.

Für humanmedizinische Zwecke ist es jedoch sinnvoller "natürliche" Testbedingungen an der menschlichen Haut zu berücksichtigen (Wärmegrad der Haut, Feuchtigkeit). Außerdem sollte der Einfluß verschiedener Hauttypen berücksichtigt werden. Zur Quantifizierung des Kaltflusses auf der menschlichen Haut kann z. B. die folgende, Methode angewandt werden.

Ein Probandenkollektiv trägt unter gleichbleibenden Bedingungen Testpflaster über 24 Stunden. Danach wird das Wandern bzw. Verrutschen der Pflaster in mm gemessen und bezüglich des Kaltflußes folgendermaßen bewertet:

### Einteilung:

| | | | |
|---|---|---|---|
| sehr gut | -5- | 0 mm | Verrutschen |
| gut | -4- | 1 mm | Verrutschen |
| mittel | -3- | 2-3 mm | Verrutschen |
| schlecht | -2- | 4-6 mm | Verrutschen |
| sehr schlecht | -1- | Pflaster schlägt Falten | |

Eine Aufgabe der vorliegenden Erfindung war es verbesserte Haft- und Bindemittel für pharmazeutische Präparationen zu entwickeln, die sich durch einen hohen Grad an Hydrophilie und somit einer guten Wasserdampfdurchlässigkeit auszeichenen. Gleichzeitig sollte eine hohe Klebkraft, aber ein geringer Kaltfluß, d. h. ein Beurteilungswert von ca. mindestens 3 der obigen Skala, erreicht werden.

Überraschenderweise wurde gefunden, daß die Aufgabe gelöst wird durch eine Haft- und Bindemittel für dermale oder transdermale Therapiesysteme bestehend aus
(a1) 55 - 99,9 Gew.-% eines (Meth)acrylatcopolymer aus strukturellen und funktionellen Monomeren, wobei die funktionellen Monomeren tertiäre oder quaternäre Aminogruppen aufweisen
(a2) 0,1 - 45 Gew -% eines säuregruppenhaltigen Acrylat- oder (Meth)acrylat Polymeren oder Copolymeren
(b) 25 - 80 Gew.-%, bezogen auf die Summe von (a1) und (a2), eines Weichmachers.

Der Erfindung liegt sie Erkenntnis zugrunde, daß die Komponenten (a1), (a2) und (b) in definierten Verhältnissen vorliegen müssen, um die gestellte Aufgabe zu lösen. Es wird angenommen, daß die erreichten vorteilhaften Effekte durch eine gegenseitigen Beeinflussung der Komponenten untereinander bewirkt werden.

### Ausführung der Erfindung

### Komponente (a1)

Die Komponente (a1) sind (Meth)acrylatcopolymere aus strukturellen und funktionellen Monomeren, wobei die funktionellen Monomeren tertiäre oder quaternäre Amino- bzw. Ammoniumgruppen aufweisen können.

Unter diese Definition fallende Copolymere sind u. a. unter dem Produktnamen EUDRAGIT® E, EUDRAGIT® RS oder EUDRAGIT® RL seit langem als Arzneimittelüberzüge bekannt.

Strukturelle Acryl- oder Methacrylat-Monomere weisen außer der Vinylfunktion keine weiteren funktionellen Reste auf. Strukturelle Acryloder Methacrylat-Monomere sind z. B. C₁- bis C₄- Alkylester der Acryloder Methacrylsäure. Bevorzugt sind Methyacrylat, Ethyacrylat, Butylacrylat, Butylmethacrylat und Methylmethacrylat.

Unter funktionellen Monomeren sind (Meth)acrylatverbindungen zu verstehen, die außer der Vinylfunktion noch weitere funktionelle Gruppen aufweisen.

Als Monomer mit funktionellen tertiären Aminogruppen wird Dimethylaminoethylmethacrylat besonders bevorzugt. Der Gehalt der funktionellen Monomere mit tertiären Ammoniumgruppen kann vorteilhafterweise zwischen 30 und 70 Gew.-%, bevorzugt 40 zwischen 60 Gew.-% liegen.

Als Monomer mit funktionellen quaternären Aminogruppen wird 2-Trimethylammoniumethlymethacrylat-Chlorid besonders bevorzugt. Der Gehalt der funktionellen Monomere mit quaternären Ammoniumgruppen liegt bevorzugt zwischen 2 und 15 Gew.-%.

Ein der Komponente (a1) entsprechendes (Meth)acrylatcopolymer mit tertiären Aminogruppen kann z. B. aus 25 Gew.-% Methylmethacrylat, 25 Gew.-% Butylmethacrylat und 50 Gew.-% Dimethylaminoethylmethacrylat aufgebaut sein (EUDRAGIT® E 100).

Ein der Komponente (a1) entsprechendes (Meth)acrylatcopolymer mit quaternären Aminogruppen , kann z. B. aus 60 Gew.-% Methylmethacrylat, 30 Gew.-% Ethylacrylat und 10 Gew.-% 2-Trimethylammoniumethlymethacrylat-Chlorid aufgebaut sein (EUDRAGIT ® RL 100).

Ein weiteres bevorzugtes der Komponente (a1) entsprechendes (Meth)acrylatcopolymer mit quaternären Aminogruppen kann z. B. aus 65 Gew.-% Methylmethacrylat, 30 Gew.-% Ethylacrylat und 5 Gew.-% 2-Trimethylammoniumethlymethacrylat-Chlorid aufgebaut sein (EUDRAGIT ® RS 100).

Die Copolymere (a) werden in an sich bekannter Weise durch radikalische Substanz-, Lösungs-, Perl- oder Emulsionspolymerisation erhalten. Sie können als extrudiertes Granulat, gemahlenes Pulver, Lösung oder Dispersion vorliegen.

### Komponente (a2)

Die Komponente (a2) sind säuregruppenhaltige Acrylat- oder (Meth)acrylat Polymere oder Copolymere.Geeignet ist z. B. Polyacrylsäure (®Carbopol). Bevorzugt sind jedoch Copolymere aus strukturellen und funktionellen (Meth)acrylat-Monomeren. Strukturelle Acryl- oder Methacrylat-Monomere sind z. B. C₁- bis C₄- Alkylester der Acryl- oder Methacrylsäure. Bevorzugt sind Methyacrylat, Ethyacrylat, Butylacrylat und Methylmethacrylat. Als Monomer mit funktionellen Säuregruppen wird Methacrylsäure besonders bevorzugt.

Ein der Komponente (a2) entsprechendes Copolymer kann z. B. aus 30 - 70 Gew.-% Ethylacrylat oder Methylmethacrylat und 70 - 30 Gew.-% Methacrylsäure aufgebaut sein.

Wesentlich für die vorliegende Erfindung ist, daß die Komponenten (a1) und (a2) in den angegebenen Verhältnissen vorliegen. Der Anteil der Komponente (a1) beträgt 55 - 99,9 Gew.-%, bevorzugt 85 - 99,9, und wird durch die Komponente (a2) zu 100 Gew.-% ergänzt. Beträgt der Anteil des säuregruppenhaltigen Copolymers (a2) weniger als 0,1 Gew.-%, so ist die Klebkraft in der Regel nicht ausreichend. Liegt der Anteil über 45 Gew.-%, so hat dies den Nachteil, daß die Verarbeitbarkeit beeinträchtigt ist. Der Anteil der Komponente (a2) liegt bevorzugt bei 15 - 0,1 Gew.-%.

Die Komponente (b), ein Weichmacher, muß zu mindestens 25 und höchstens 80 Gew.-%, bevorzugt 30 - 60 Gew.-%, bezogen auf die Summe der Komponenten (a1) und (a2) vorhanden sein. Bei weniger als 25 Gew.-% Weichmacher wird in der Regel keine ausreichende Hauthaftung erreicht. Liegt der Anteil über 80 Gew.-% ist in der Regel das Wirkstoffabgabeverhalten nur schwer steuerbar.

Als Weichmacher geeignete Stoffe haben in der Regel ein Molekulargewicht zwischen 100 und 20 000 und enthalten eine oder mehrere hydrophile Gruppen im Molekül, z. B. Hydroxyl- Ester- oder Aminogruppen. Beispiele geeigneter Weichmacher sind Citronensäurealkylester, Glycerinester, Phthalsäurealkylester, Sebacinsäurealkylester, Sucroseester, Sorbitanester, Dibutylsebacat und Polyethylenglykole 4000 bis 20.000. Bevorzugte Weichmacher sind Triethylcitrat und Acetyltriethylcitrat.

Der Weichmacherzusatz erlaubt die Anpassung physikalischer Eigenschaften an die Erfordernisse der einzelnen Arzneiformen, sodaß bei Raum- bzw. Körpertemperatur ausreichende Haftkräfte erreicht werden.

Außerdem können die Weichmacher in den angegebenen Verhältnissen die Schmelzviskosität der eingesetzten Polymere im flüssigen Zustand vorteilhaft erniedrigen.
Bei Raumtemperatur sind erweichende Effekte zu erkennen. Einflüsse auf das Freigabeverhalten eingebetteter Wirkstoffe sind möglich.

Variationen der Zusammensetzung ermöglichen es gegebenenfalls unerwünschte Effekte von arzneiformbedingten Zusätzen auszugleichen. Die erfindungsgemäßen Haft- und Bindemittel können optional weitere Zusätze in geringen Mengen enthalten, wenn es die spezielle Formulierung erfordert: Neutrale Polymere, Tackifyer, Stabilisatoren, Farbstoffe, Antioxidantien, Netzmittel, Porenbildner, Feuchthaltemittel, komplexierende Mittel u.a..

### Herstellungsverfahren:

Die Herstellung des Bindemittels hängt von der eingesetzten Form des Polymeren ab: Festsubstanzen können direkt eingesetzt werden durch Mischen mit den Zusatzstoffen in geeigneten Mischern, Knetern oder Extrudern, die heizbare und ggf. evakuierbar sind. Der Extruder ist ein - oder bevorzugt doppelschneckig, um geeignete Misch- und Transporteigenschaften zu erreichen.

Die Verarbeitungstemperatur richtet sich nach den Schmelzeigenschaften der Materialien und liegt vorzugsweise zwischen 20°C und 200°C. Einschränkende Faktoren sind die thermische Stabilität der Einsatzstoffe. Feste Zuschlagstoffe können vor der Extrusion mit den Polymer gemischt werden. Flüssige Zuschlagstoffe werden auf etwa der halben Extrusionsstrecke der Schmelze zugesetzt und bewirken eine Viskositätserniedrigung und Temperaturabsenkung.

Polymerlösungen oder Dispersionen werden mit den Zusatzstoffen versetzt, so daß diese sich auflösen oder suspendiert werden. Aus diesen Lösungen, Dispersionen oder Suspensionen erhält man das Bindemittel durch Trocknen zu dünnen Filmschichten.

### Verarbeitung:

Beschichtung, Granulation, Umhüllung oder Einbettung erfolgen mittels organischer Lösung oder wäßriger Dispersion von geeigneten Hilfsstoffen.
Die Verwendung von Schmelzen beschränkt sich auf Substanzen mit definierten Schmelzpunkten im Bereich der Verarbeitungstemperaturen. Üblicherweise benötigt man niedrige Schmelzviskositäten für die Verarbeitung.

In einer Verfahrensvariante wird das erfindungsgemäße, feste Haft- und Bindemittel mit den Pulvern gemischt und mit einem geeigneten Lösungsmitten gemischt oder gemeinsam aufgeschmolzen.

Bevorzugt aus Lösung bzw. Suspension oder direkt aus der Schmelze erhält man durch Ausstreichen auf flächige Träger z.B. Folien, Gewebe oder Vliese, nach Trocknung oder Abkühlung Haftschichten, die das System auf der Haut fixieren und wegen der Hydrophilie besonders gut verträglich sind. Die Beschichtung erfolgt im Labor diskontinuierlich mittels einer Rakel und im Technikum und Produktion kontinuierlich mittels Rollrakel oder Walzenauftrag. Direkt nach der Beschichtung fügt man eine schwach haftende, oft silikonisierte Deckfolie hinzu, die vor der Anwendung entfernt wird.

Die erhaltenen Agglomerate oder Haftschichten können für die Anwendung zu Arzneiformen weiterverarbeitet werden. Dabei ist es möglich Arzneistoffe schon während der Herstellung des Haft- und Bindemittels einzuarbeiten. Diese Wirkstoffe sind dann in partikulärer oder gelöster Form fixiert. Eine Beeinflussung der Wirkstoffabgabe durch das Haft- und Bindemittel ist möglich und kann für die Formulierung von Arzneiformen ausgenutzt werden.

### Formulierung der Arzneiformen

Die im Sinne der Erfindung eingesetzten Arzneistoffe sind dazu bestimmt, am oder im menschlichen oder tierischen Körper Anwendung zu finden, um
1. Krankheiten, Leiden, Körperschäden oder krankhafte Beschwerden zu heilen, zu lindern, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

Gebräuchliche Arzneistoffe sind Nachschlagewerken, wie z.B. der Roten Liste oder dem Merck Index zu entnehmen.

Erfindungsgemäß können alle Wirkstoffe eingesetzt werden, die die gewünschte therapeutische Wirkung im Sinne der obigen Definition erfüllen und eine ausreichende thermische Stabilität besitzen.

Wichtige Beispiele (Gruppen und Einzelsubstanzen) ohne Anspruch auf Vollständigkeit sind folgende:
Analgetika,
Antiallergika, Antiarrhythmika
Antibiotika, Chemotherapeutika, Antidiabetika, Antidote,
Antiepileptika, Antihypertonika, Antihypotonika,
Antikoagulantia, Antimykotika, Antiphlogistika,
Betarezeptorenblocker, Calciumantagonisten und ACE-Hemmer,
Broncholytika/Antiasthmatika, Cholinergika, Corticoide (Interna),
Dermatika, Diuretika, Enzyminhibitoren, Enzympräparate und
Transportproteine,
Expectorantien, Geriatrika, Gichtmittel, Grippemittel,
Hormone und deren Hemmstoffe, Hypnotika/Sedativa, Kardiaka, Lipidsenker,
Nebenschilddrüsenhormone/Calciumstoffwechselregulatoren,
Psychopharmaka, Sexualhormone und ihre Hemmstoffe,
Spasmolytika, Sympatholytika, Sympathomimetika, Vitamine,
Wundbehandlungsmittel, Zytostatika.

Arzneiformen lassen sich aus den erfindungsgemäß hergestellten Zwischenstufen durch übliche Verabeitungstechniken herstellen.

**Das Haft- und Bindemittels kann zur Herstellung eines transdermalen Therapiesystems, in dem ein pharmazeutischer Wirkstoff durch Beschichtung oder durch Sprühen oder Bestreichen von Lösungen, Dispersionen, Suspensionen oder Schmelzen eines Haft- und Bindemittels und anschließendes Trocknen bzw. Abkühlen eingearbeitet wird, verwendet werden.**
**Als pharmazeutischer Wirkstoffe können Nicotin, Glyceroltrinitrat, Scopolamin, Clonidin, Fentanyl, Östradiol, Testosteron, Oxibutynin, Diclophenac, Ibuprofen, Ketoprofen, Diltiazem, Propranolol, Albuterol, Alprazolam, Amethocaine, Atenolol, Benzoporphyrin, Buprenorphine, Calcitonin, Dithranol, Diphencypron, diverse Peptide, Eptazocine, Ethinylöstradiol, Methrotrexat oder Naloxon verwendet werden.**

Mit dem Haft- und Bindemittel bestrichene Träger liegen in der Regel auf Rollen vor, geschützt durch Deckfolien (release liner). Aus diesen Bahnen werden einzelne Pflaster der erforderlichen Größe'geschnitten oder gestanzt und einzeln verpackt.
Das Beschichten flächiger Träger mit polymerhaltigen Flüssigkeiten ist z. B. in Mass, J. und Schmidt, H. : Coating Technology for Transdermal Drug Delivery Systems, Medical Device Technology, Ausgabe 3/41990, S. 46 - 50, beschrieben.

Für die Applikation relevante Eigenschaften, geforderte Tests und Spezifikationen sind in Arzneibüchern aufgelistet.

Details sind den gängigen Lehrbüchern zu entnehmen, z.B.:
- Voigt, R. (1984): Lehrbuch der pharmazeutischen Technologie; Verlag Chemie Weinheim - Beerfield Beach/Florida - Basel.
- Sucker, H., Fuchs, P., Speiser, P. : Pharmazeutische Technologie, Georg Thieme Verlag Stuttgart (1991), insbesondere Kapitel 15 und 16, S. 626 - 642.
- Gennaro, A.,R. (Editor), Remington's Pharmaceutical Sciences, Mack Publishing Co., Easton Pennsylvania (1985), Chapter 88, S. 1567 - 1573.
- Heilmann, K.: Therapeutische Systeme, Ferdinand Euler Verlag, Stuttgart, S. 52 -57.
- Brandau, R. und Lippold, B. H. (1982): Dermal and Transdermal Absorption. Wissenschaftliche Verlagsgesellschaft mbH, Stuttgart, S. 171 - 200.

### BEISPIELE

### Beispiel 1:

### Haftschicht für dermale und transdermale Therapiesysteme aus organischer Lösung

40 g eines Copolymers (a1) aus 25 Gew.-% Methylmethacrylat, 25 Gew.-% Butylmethacrylat und 50 Gew.-% Dimethylaminoethylmethacrylat (Handelsname ®EUDRAGIT E 100) werden in einer Mischung aus 24 g Aceton, 12 g Ethanol und 3 g Isopropanol gelöst. In diese Lösung werden 20 g des Weichmachers Acetyltriethylcitrat (Komponente b) und bei hoher Rührgeschwindigkeit 0,1 g eines Copolymers (a2) aus 50 Gew.-% Methylmethacrylat und 50 Gew.-% Methacrylsäure (Handelsname ® EUDRAGIT L 100) als 20 %ige, ethanolische Lösung eingearbeitet. Auf einer 50 µm dicken Aluminiumfolie wird eine 200 µm dicke Schicht des Klebers bei 60°C 10 min. getrocknet. Aus der Folie stanzt man runde Pflaster mit einem Durchmesser von 43 mm. Diese Muster kleben fest auf der menschlichen Haut und lassen sich rückstandsfrei abziehen. Auf einer 50 µm dicken Aluminiumfolie wird eine 300 um dicke Schicht des Klebers mittels einer Rackel bei 120°C ausgezogen und abgekühlt. Die Haftschicht hat eine Klebkraft von 63 [N/50 mm] gemessen in Anlehnung an die Peel method, Ph Eur. II. zeigt.

### Beispiel 2:

### Haftschicht für dermale und transdermale Therapiesysteme aus einer Schmelze

Der Schmelzprozeß wird in einem Meßkneter durchgeführt. Der Mischtrog wird auf 120°C vorgeheizt. Bei dieser Temperatur, die während der gesamten Prozeßdauer gehalten wird, werden portionsweise 100 g ®EUDRAGIT E 100, 50 g Triethylcitrat und 1,5 g ®EUDRAGIT L 100 eingearbeitet. Die Rührgeschwindigkeit beträgt 25 U/min. Diese Lösung wird wie in Beispiel 3 beschichtet. Aus der Folie stanzt man runde Pflaster mit einem Durchmesser von 43 mm. Diese Muster kleben fest auf der menschlichen Haut und lassen sich rückstandsfrei abziehen. Auf einer 50 µm dicken Aluminiumfolie wird eine 300 µm dicke Schicht des Klebers mittels einer Rackel bei 120°C ausgezogen und abgekühlt. Die Haftschicht hat eine Klebkraft von 58 [N/50 mm] gemessen in Anlehnung an die Peel method, Ph Eur. II. zeigt.

### Beispiel 3

### Haftschicht für dermale und transdermale Therapiesysteme aus einer Schmelze

In einem heizbaren Kneter mit Doppelmantel wird der Mischtrog auf 120°C vorgeheizt. Bei dieser Temperatur, die während der gesamten Prozeßdauer gehalten wird, werden portionsweise 400 g ®EUDRAGIT E 100, 160 g Triethylcitrat und 40 g ®EUDRAGIT L 100-55 (Copolymer (a2) aus 50 Gew.-% Ethylacrylat und 50 Gew.-% Methacrylsäure) eingearbeitet. Die Rührgeschwindigkeit beträgt
25 U/min. Es entsteht eine klare, leicht gelblich gefärbte Schmelze, die mittels einer Rakel auf einem beheizten Tisch auf Aluminiumfolie zu einer ca. 150 µm Schicht ausgezogen wird. Aus der Folie stanzt man runde Pflaster mit einem Durchmesser von 43 mm. Diese Muster kleben fest auf der menschlichen Haut und lassen sich rückstandsfrei abziehen.
Auf einer 50 µm dicken Aluminiumfolie wird eine 300 µm dicke Schicht des Klebers mittels einer Rackel bei 120°C ausgezogen und abgekühlt. Die Haftschicht hat eine Klebkraft von 69 [N/50 mm] gemessen in Anlehnung an die Peel method, Ph Eur. II. zeigt.

### Beispiel 4:

### Haftschicht für dermale und transdermale Therapiesysteme aus einer Schmelze

In einem heizbaren Kneter mit Doppelmantel wird der Mischtrog auf 120°C vorgeheizt. Bei dieser Temperatur, die während der gesamten Prozeßdauer gehalten wird, werden portionsweise 180,0 g ®EUDRAGIT RS 100, 123,6 g Triethylcitrat und 120,6 g ®EUDRAGIT L 100 (Copolymer (a2) aus 50 Gew.-% Methylmethacrylat und 50 Gew.-% Methacrylsäure) eingearbeitet. Die Rührgeschwindigkeit beträgt 25 U/min. Es entsteht eine gelblichweiß gefärbte Schmelze, die mittels einer Rakel auf einem beheizten Tisch auf Aluminiumfolie zu einer ca. 300 µm Schicht ausgezogen wird. Aus der Folie stanzt man runde Pflaster mit einem Durchmesser von 43 mm, entsprechend 15cm². Diese Muster kleben fest auf der menschlichen Haut und lassen sich rückstandsfrei abziehen.

Auf einer 50 µm dicken Aluminiumfolie wird eine 300 µm dicke Schicht des Klebers mittels einer Rackel bei 120°C ausgezogen und abgekühlt. Die Haftschicht hat eine Klebkraft von 53 [N/50 mm] gemessen in Anlehnung an die Peel method, Ph Eur. II. zeigt.

### Vergleichsbeispiele

Präparationen, bei denen die Mengenanteile in nicht erfindungsgemäßer Weise variiert wurden (Vergleiche 1 - 5) wurden in Bezug auf Hydrophilie Klebkraft und Kaltfluß geprüft. Die Ergebnisse sind in Tabelle 1 im Vergleich zu den Beispielen 1 - 4 zusammengefaßt.

**Tabelle 1:**

| | a1 Gew.-% | a2 Gew.-% | b : a1 + a2 | Hydrophilie WDD DIN 53 122 | Klebkraft (N/50 mm) | Kaltfluß* |
|---|---|---|---|---|---|---|
| B1 | 99,75(E) | 0,25 L100 | 49,9 ATEC | 470 | 63 | 3 |
| B2 | 98,50(E) | 1,50 L100 | 49,**3** TEC | 510 | 58 | 3 |
| 83 | **90,9** (E) | **9,1** L55 | 36,**4** TEC | 590 | 69 | 5 |
| B4 | **59,9** (RS) | 40,**1** L100 | 41,**1** TEC | 390 | 52 | 3 |
| Vgl 1 | 100,00 (RS) | -- | 20,0 TEC | 250 | <10 | --** |
| Vgl 2 | 100,00 (RL) | -- | 50,0 TEC | --** | <10 | --** |
| Vgl. 3 | 90,00(E) | 10,0 L100 | 20,0 TEC | 540*** | < 10 | --** |
| Vgl 4 | 50,00(RS) | 50,0 L100 | 50,0 TEC | 410 | 53 | 2 |
| Vgl 5 | 100,00 (RL) | -- | 80,0 TEC | --** | <10 | --** |

| | | | | | | |
|---|---|---|---|---|---|---|
| *Skalierung von 1 = sehr schlecht bis 5 = sehr gut | | | | | | |
| ** nicht bestimmt, da Klebkraft zu gering | | | | | | |
| *** geschätzt **E** = Copolymer aus 25 Gew.-% Methylmethacrylat, 25 Gew.-% Butylmethacrylat und 50 Gew.-% Dimethylaminoethyimethacrylat; (EUDRAGIT® E 100, Röhm GmbH, D-64293 Darmstadt, Deutschland). **RS** = Copolymer aus 65 Gew.-% Methylmethacrylat, 30 Gew.-% Ethylacrylat und 5 Gew.-% 2-Trimethylammoniumethlymethacrylat-Chlorid (EUDRAGIT® RS, Röhm GmbH, D-64293 Darmstadt, Deutschland). **RL** = Copolymer aus 60 Gew.-% Methylmethacrylat, 30 Gew.-% Ethylacrylat und 10 Gew.-% 2-Trimethylammoniumethlymethacrylat-Chlorid (EUDRAGIT® RL, Röhm GmbH, D-64293 Darmstadt, Deutschland). **ATEC:** Acetyltriethylcitrat; **TEC**=Triethylcitrat **L 100** = Copolymer aus 50 Gew.-% Methylmethacrylat und 50 Gew.-% Methacrylsäure (EUDRAGIT® L100). **L55** = Copolymer aus 50 Gew.-% Ethylacrylat und 50 Gew.-% Methacrylsäure (EUDRAGIT® L100-55) | | | | | | |

## Patentansprüche

1. Haft- und Bindemittel für dermale oder transdermale Therapiesysteme bestehend aus den Komponenten
(a1) 55 - 99,9 Gew.-% eines (Meth)acrylatcopolymers aus strukturellen und funktionellen Monomeren, wobei die funktionellen Monomeren tertiäre oder quaternäre Aminogruppen aufweisen
(a2) 0,1 - 45 Gew.-% eines säuregruppenhaltigen Acrylat- oder (Meth)acrylat Polymeren oder Copolymeren und
(b) 25 - 80 Gew.-%, bezogen auf die Summe von (a1) und (a2), eines Weichmachers.

2. Haft- und Bindemittel nach Anspruch 1, **dadurch gekennzeichnet, daß** die Menge des Weichmachers (b) 30 - 60 Gew.-%, bezogen auf die Summe von (a1) und (a2) beträgt.

3. Haft- und Bindemittel nach Anspruch 1, **dadurch gekennzeichnet, daß** die Komponente (a2) ein (Meth)acrylat-Copolymer aus 30 - 70 Gew.-% Ethylacrylat oder Methylmethacrylat und 70 bis 30 Gew.-% Methacrylsäure ist.

4. Verwendung eines Haft- und Bindemittels gemäß Anspruch 1 zur Herstellung eines transdermalen Therapiesystems, in dem ein pharmazeutischer Wirkstoff durch Beschichtung oder durch Sprühen oder Bestreichen von Lösungen, Dispersionen, Suspensionen oder Schmelzen eines Haft- und Bindemittels und anschließendes Trocknen bzw. Abkühlen eingearbeitet wird.

5. Verwendung nach Anspruch 4, **dadurch gekennzeichnet, daß** der pharmazeutische Wirkstoff Nicotin, Glyceroltrinitrat, Scopolamin, Clonidin, Fentanyl, Östradiol, Testosteron, Oxibutynin, Diclophenac, Ibuprofen, Ketoprofen, Diltiazem, Propranolol, Albuterol, Alprazolam, Amethocaine, Atenolol, Benzoporphyrin, Buprenorphine, Calcitonin, Dithranol, Diphencypron, diverse Peptide, Eptazocine, Ethinylöstradiol, Methotrexat oder Naloxon ist.

## Claims

1. Adhesives and binders for dermal or transdermal therapeutic systems consisting of the components
(a1) 55-99.99 % by weight of a (meth)acrylate copolymer obtained from structural and functional monomers, the functional monomers having tertiary or quaternary amino groups
(a2) 0.1 - 45 % by weight of an acrylate or (meth)acrylate polymer or copolymer containing acid groups and
(a3) 25 - 80 % by weight, based on the total of (a1) and (a2), of a plasticiser.

2. Adhesives and binders according to claim 1, **characterised in that** the quantity of the plasticiser (b) is 30 - 60% by weight, based on the total of (a1) and (a2).

3. Adhesives and binders according to claim 1, **characterised in that** component (a2) is a (meth)acrylate copolymer comprising 30-70% by weight of ethyl acrylate or methyl methacrylate and 70 to 30% by weight of methacrylic acid.

4. Use of an adhesive and binder according to claim 1 for preparing a transdermal therapeutic system in which a pharmaceutical active substance is incorporated by coating or spraying or spreading solutions, dispersions, suspensions or melts of an adhesive and binder and subsequently drying or cooling.

5. Use according to claim 4, **characterised in that** the pharmaceutical active substance is nicotine, glycerol trinitrate, scopolamine, clonidine, fentanyl, oestradiol, testosterone, oxibutynine, diclophenac, ibuprofen, ketoprofen, diltiazem, propranolol, albuterol, alprazolam, amethocaine, atenolol, benzoporphyrin, buprenorphine, calcitonin, dithranol, diphencypron, various peptides, eptazocine, ethynyl oestradiol, methotrexate or naloxone.

## Revendications

1. Adhésif et liant pour systèmes thérapeutiques à usage dermique et transdermique constitué des composants :
(a1) 55 à 99,9 % en poids d'un copolymère de (méth)acrylate constitué de monomères structuraux fonctionnels, les monomères fonctionnels présentant des groupes amino tertiaires ou quaternaires ;
(a2) de 0,1 à 45 % en poids d'un polymère ou copolymère d'acrylate ou de (méth)acrylate contenant des groupes acides ;
(b) de 25 à 80 % en poids, par rapport à la somme d'(a1) et (a2), d'un plastifiant.

2. Adhésif et liant selon la revendication 1,
**caractérisés en ce que**
la quantité de plastifiant (b) s'élève à 30 à 60 % en poids par rapport à la somme de (a1) et (a2).

3. Adhésif et liant selon la revendication 1,
**caractérisés en ce que**
le composant (a2) est un copolymère de méthacrylate constitué de 30 à 70 % en poids d'acrylate d'éthyle ou de méthacrylate de méthyle et de 70 à 30 % en poids d'acide méthacrylique.

4. Utilisation d'un adhésif et liant selon la revendication 1,
pour la fabrication d'un système thérapeutique par voie transdermique dans lequel on intègre une substance active pharmaceutique par enduction ou par pulvérisation ou revêtement de solutions, dispersions, suspensions ou masses fondues d'un adhésif et liant puis séchage ou selon les cas refroidissement.

5. Utilisation selon la revendication 4,
**caractérisée en ce que**
la substance active pharmaceutique est la nicotine, le trinitrate de glycérol, la scopolamine, la clonidine, le fentanyle, l'oestradiol, la testostérone, l'oxybutynine, le diclophénac, l'ibuprofène, le kétoprofène, le diltiazem, le propanolol, l'albutérol, l'alprazolam, l'améthocaïne, l'aténolol, la benzoporphyrine, la buprénorphine, la calcitonine, le dithranol, la diphéncyprone, divers peptides, l'eptazocine, l'éthynyloestradiol, le méthotrexate ou la naxolone.
